# EUROPEAN PATENT APPLICATION

(11) **EP 4 846 971 A1**
(43) Date of publication of application: **02.12.2026**
(21) Application number: 26206267.2
(22) Date of filing: 21.09.2026
(51) Int. Cl.: A61B 34/20, A61B 90/00, A61C 9/00, A61B 34/10, A61C 1/08

(54) **NAVIGATION APPARATUS, DEVICE AND SYSTEM**

(30) Priority: 28.09.2025 CN 202511405350
(71) Applicant: Shining 3D Tech Co., Ltd., Hangzhou, Zhejiang 311258 (CN)
(72) Inventor: FU, Xiaopeng, Hangzhou, 311258 (CN); ZHAO, Xiaobo, Hangzhou, 311258 (CN); CHEN, Xiaojun, Hangzhou, 311258 (CN); LI, Zexue, Hangzhou, 311258 (CN)
(74) Representative: Dai, Simin

(57) **Abstract**

Embodiments of the present disclosure provide a navigation method, apparatus, device, and system. Local data of a target oral cavity may be reconstructed from a real-time image of the target oral cavity captured by a scanning camera on an intraoral scanning device, an alignment relationship between the local data and a pre-constructed reference model involving the target oral cavity may be determined, a relative pose relationship between a dental instrument and the intraoral scanning device may be determined based on an image of a marker on the intraoral scanning device captured by a tracking camera on the dental instrument and/or an image of a marker on the dental instrument captured by a tracking camera on the intraoral scanning device, and the reference model and a virtual dental instrument may be displayed on an interactive interface based on the alignment relationship and the relative pose relationship.

## Description

### TECHNICAL FIELD

Embodiments of the present disclosure relate to the field of oral medical technologies, and in particular, to a navigation method, a navigation apparatus, an electronic device, and a navigation system.

### BACKGROUND

During implant navigation surgery, it is usually necessary to track a dental instrument in real time by means of a large external tracking device provided with an optical tracker to determine a real-time pose of the dental instrument. Since the external tracking device is usually bulky and occupies a large space, the working space available to a medical staff is limited. Moreover, when the external tracking device is used to track the dental instrument in real time, it is usually necessary to use the external tracking device to capture markers disposed on teeth of the patient or the dental instrument. The markers are highly susceptible to occlusion, which prevents the external tracking device from capturing the markers, resulting in tracking failure.

### SUMMARY

Embodiments of the present disclosure provide a navigation method, a navigation apparatus, an electronic device, and a navigation system.

According to a first aspect of the embodiments of the present disclosure, there is provided a navigation method, including:
acquiring a real-time image of a target oral cavity captured by a scanning camera on an intraoral scanning device, and reconstructing local data of the target oral cavity from the real-time image;
determining an alignment relationship between the local data and a pre-constructed reference model involving the target oral cavity;
determining a relative pose relationship between a dental instrument and the intraoral scanning device based on an image of a marker on the intraoral scanning device captured by a tracking camera on the dental instrument and/or an image of a marker on the dental instrument captured by a tracking camera on the intraoral scanning device, where the scanning camera and the tracking camera on the intraoral scanning device are mounted at different positions;
displaying the reference model and a virtual dental instrument on an interactive interface based on the alignment relationship between the local data and the reference model and the relative pose relationship between the dental instrument and the intraoral scanning device, so as to indicate a pose of the dental instrument in the target oral cavity by a pose of the virtual dental instrument in the reference model.

According to a second aspect of the embodiments of the present disclosure, there is provided a navigation system, including a dental instrument, an intraoral scanning device, and a data processing apparatus communicatively connected to the intraoral scanning device; where the intraoral scanning device is configured to capture a real-time image of a target oral cavity by a scanning camera, and send the real-time image to the data processing apparatus; and
the data processing apparatus is configured to: acquire the real-time image, and reconstruct local data of the target oral cavity from the real-time image; determine an alignment relationship between the local data and a pre-constructed reference model involving the target oral cavity; determine a relative pose relationship between the dental instrument and the intraoral scanning device based on an image of a marker on the intraoral scanning device captured by a tracking camera on the dental instrument and/or an image of a marker on the dental instrument captured by a tracking camera on the intraoral scanning device, where the scanning camera and the tracking camera on the intraoral scanning device are mounted at different positions; and display the reference model and a virtual dental instrument on an interactive interface based on the alignment relationship between the local data and the reference model and the relative pose relationship between the dental instrument and the intraoral scanning device, so as to indicate a pose of the dental instrument in the target oral cavity by a pose of the virtual dental instrument in the reference model.

According to a third aspect of the embodiments of the present disclosure, there is provided a navigation apparatus, including:
an acquisition module, configured to acquire a real-time image of a target oral cavity captured by a scanning camera on an intraoral scanning device, and reconstruct local data of the target oral cavity from the real-time image;
an alignment module, configured to determine an alignment relationship between the local data and a pre-constructed reference model involving the target oral cavity;
a relative pose relationship determination module, configured to determine a relative pose relationship between a dental instrument and the intraoral scanning device based on an image of a marker on the intraoral scanning device captured by a tracking camera on the dental instrument and/or an image of a marker on the dental instrument captured by a tracking camera on the intraoral scanning device, where the scanning camera and the tracking camera on the intraoral scanning device are mounted at different positions; and
a display module, configured to display the reference model and a virtual dental instrument on an interactive interface based on the alignment relationship between the local data and the reference model and the relative pose relationship between the dental instrument and the intraoral scanning device, so as to indicate a pose of the dental instrument in the target oral cavity by a pose of the virtual dental instrument in the reference model.

According to a fourth aspect of the embodiments of the present disclosure, there is provided an electronic device, including at least one processor, at least one memory, and computer instructions stored in the memory and executable by the processor, where the processor, when executing the computer instructions, implements the method mentioned in the above first aspect.

According to a fifth aspect of the embodiments of the present disclosure, there is provided a computer-readable storage medium, having computer instructions stored therein, where the computer instructions, when executed, cause the method mentioned in the above first aspect to be implemented.

In the embodiments of the present disclosure, the intraoral scanning device may be used to track the dental instrument instead of an external tracking device. For example, the tracking camera may be disposed on the intraoral scanning device and/or the dental instrument, and the tracking camera of the intraoral scanning device is used to capture the image of the marker on the dental instrument, and/or the tracking camera of the dental instrument is used to capture the image of the marker on the intraoral scanning device, and the real-time relative pose between the two is determined based on the captured images. Meanwhile, during the implant surgery, the scanning camera on the intraoral scanning device may be used to scan the target oral cavity at the same time, the local data of the target oral cavity is reconstructed from the real-time image captured by the intraoral scanning device in a single scan, the alignment relationship between the local data and the pre-constructed reference model involving the target oral cavity is determined, and the reference model and the virtual dental instrument are displayed on the interactive interface based on the alignment relationship between the local data and the reference model and the relative pose relationship between the dental instrument and the intraoral scanning device, so as to indicate the pose of the dental instrument in the target oral cavity by the pose of the virtual dental instrument in the reference model.

By replacing the external tracking device with the intraoral scanning device, which is usually a portable device with a small volume, the space occupation can be greatly reduced compared with the external tracking device, thereby providing a larger working space for the medical staff. Meanwhile, the intraoral scanning device and the dental instrument both need to be placed in the target oral cavity for operation, and are positioned close to each other, thereby reducing tracking failure caused by occlusion of the markers on the intraoral scanning device or the dental instrument. In addition, with the navigation method of the present disclosure, there is no need to additionally provide markers in the target oral cavity, which can avoid tracking loss caused by detachment or occlusion of the markers, and can also reduce patient discomfort.

It should be understood that the above general description and the following detailed description are merely exemplary and explanatory, and are not intended to limit the embodiments of the present disclosure.

### BRIEF DESCRIPTION OF DRAWINGS

The accompanying drawings herein, which are incorporated into and constitute a part of this specification, illustrate embodiments consistent with embodiments of the present disclosure and, together with the specification, serve to explain the technical solutions of the embodiments of the present disclosure.
FIG. 1 is a schematic diagram of an external tracking device in the related art.
FIG. 2A is a schematic diagram of a navigation system according to an embodiment of the present disclosure.
FIG. 2B is a schematic diagram of a navigation system according to another embodiment of the present disclosure.
FIG. 3 is a flowchart of a navigation method according to an embodiment of the present disclosure.
FIG. 4 is a schematic diagram of determining a relative pose relationship between a dental instrument and an intraoral scanning device based on an image of the dental instrument captured by the intraoral scanning device according to an embodiment of the present disclosure.
FIG. 5 is a schematic diagram of determining a relative pose relationship between a dental instrument and an intraoral scanning device based on an image of the intraoral scanning device captured by the dental instrument according to an embodiment of the present disclosure.
FIG. 6 is a schematic diagram of determining a relative pose relationship between a dental instrument and an intraoral scanning device based on an image of the dental instrument captured by the intraoral scanning device and an image of the intraoral scanning device captured by the dental instrument according to an embodiment of the present disclosure.
FIG. 7 is a schematic diagram of a calibration plate according to an embodiment of the present disclosure.
FIG. 8A is a schematic diagram of a position of a tracking camera on a dental instrument according to an embodiment of the present disclosure.
FIG. 8B is a schematic diagram of a position of a tracking camera on an intraoral scanning device according to an embodiment of the present disclosure.
FIG. 9A is a schematic diagram of aligning a single-frame intraoral scan data with a three-dimensional medical image model according to an embodiment of the present disclosure.
FIG. 9B is a schematic diagram of aligning local data with a global three-dimensional tooth model according to an embodiment of the present disclosure.
FIG. 10 is a schematic diagram of a logical structure of a navigation apparatus according to an embodiment of the present disclosure.
FIG. 11 is a schematic diagram of a logical structure of an electronic device according to an embodiment of the present disclosure.

### DETAILED DESCRIPTION

Reference will now be made in detail to exemplary embodiments, examples of which are illustrated in the accompanying drawings. When the following description refers to the accompanying drawings, unless otherwise indicated, the same numbers in different accompanying drawings indicate the same or similar elements. The implementations described in the following example embodiments do not represent all implementations consistent with the embodiments of the present disclosure. On the contrary, they are merely examples of apparatuses and methods consistent with some aspects of the embodiments of the present disclosure as detailed in the appended claims.

Terms used in the embodiments of the present disclosure are merely intended to describe specific embodiments, but are not intended to limit the embodiments of the present disclosure. As used in the embodiments of the present disclosure and the appended claims, the singular forms "a", "an", and "the" are intended to include the plural forms as well, unless the context clearly indicates otherwise. It should also be understood that the term "and/or" used herein refers to and includes any or all possible combinations of one or more of the associated listed items. In addition, the term "at least one" herein means any one of a plurality or any combination of at least two of a plurality.

It should be understood that, although the terms first, second, third, and the like may be used in the embodiments of the present disclosure to describe various information, the information should not be limited by these terms. These terms are only used to distinguish the same type of information from each other. For example, without departing from the scope of the embodiments of the present disclosure, first information may also be referred to as second information, and similarly, second information may also be referred to as first information. Depending on the context, the word "if" as used herein may be interpreted as "when ..." or "upon ..." or "in response to determining".

To enable a person skilled in the art to better understand the technical solutions in the embodiments of the present disclosure, and make the above objectives, features, and advantages of the embodiments of the present disclosure clearer and easier to understand, the technical solutions in the embodiments of the present disclosure will be further described in detail below with reference to the accompanying drawings.

Surgical navigation includes implant navigation. Taking implant navigation as an example, implant navigation is an assistive technology used in an oral implant surgery, which uses advanced computer technology, imaging technology, and navigation device to help dentists more accurately determine the position, direction, and depth of an implant during an implant surgery, thereby improving the success rate and safety of the implant surgery.

The implant navigation technology includes two core steps of preoperative planning and intraoperative navigation. The preoperative planning refers to acquisition of a reference model involving an oral cavity of the patient before surgery. For example, a three-dimensional CBCT (cone beam computed tomography) model involving the oral cavity of the patient is acquired using CBCT, and a three-dimensional CT (computed tomography) model involving the oral cavity of the patient is acquired using CT. The reference model may involve important structures such as alveolar bones, nerves, and blood vessels. Then, the implantation position, angle, and depth of the implant may be simulated in computer software based on the reference model to help the dentists evaluate the feasibility and safety of the surgical plan, so as to design the most suitable implantation plan. The intraoperative navigation refers to, the process during an actual implant surgery in which the dentist may use an external tracking apparatus in a navigation system to track a position and movement trajectory of a dental instrument (e.g., a tool used for drilling and grinding tooth structures and preparing implant cavities) in real time. The dental instrument and a pre-constructed reference model involving the oral cavity of the patient are then represented in the same coordinate system, so that the reference model and a virtual dental instrument can be simultaneously displayed on an interactive interface based on the poses of the two in the same coordinate system. This allows the dentist to know the current real-time pose of the dental instrument in the patient's actual oral cavity based on the pose of the virtual dental instrument in the reference model, thereby facilitating the dentist to perform the surgery.

During the intraoperative navigation, it is critical to unify coordinate systems of the dental instrument and the reference model in order to enable the dentist to intuitively see the current real-time pose of the dental instrument in the target oral cavity. Accurately determining the poses of the two in the same coordinate system can facilitate the dentist to understand the current pose of the dental instrument in the oral cavity and the relative positions of the dental instrument to structures such as blood vessels and nerves, and can provide accurate reference information for the dentist to perform the surgery.

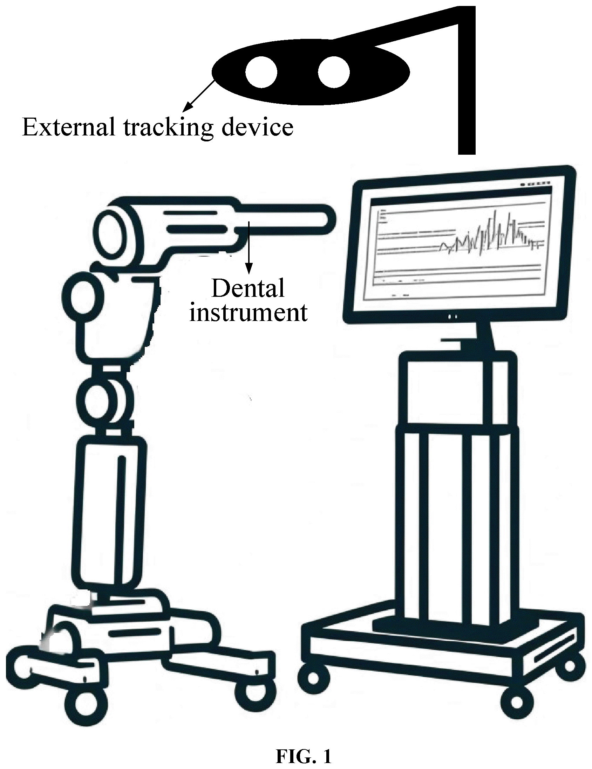

In the related art, in order to represent the dental instrument and the reference model involving the oral cavity of the patient in the same coordinate system, it is usually necessary to fix a marker in the oral cavity of the patient, and then the oral cavity of the patient may be scanned by using CBCT, CT, etc., to obtain a reference model carrying the marker. As shown in FIG. 1, during the surgery, it is usually necessary to track the dental instrument in real time by means of an external tracking device, where the external tracking device is usually placed next to a dental chair, and the external tracking device is usually provided with an optical tracker. During the implant surgery, the optical tracker on the external tracking device can track the marker fixed in the oral cavity of the patient and the marker on the dental instrument, and the dental instrument and the reference model can be represented in the same coordinate system based on the tracking data, so as to simultaneously display the reference model and the virtual dental instrument on the interactive interface based on the poses of the two in the same coordinate system, so that the dentist can learn the current real-time pose of the dental instrument in the real oral cavity of the patient based on the pose of the virtual dental instrument in the reference model, thereby facilitating the dentist to perform the surgery.

The above navigation method has many problems. Firstly, the external tracking device is often bulky and requires a large space, while a working area next to the dental chair is very narrow. If a large-sized external tracking device is to be placed next to the dental chair, it may interfere with the working area available to the medical staff, resulting in limited movement space. Secondly, during the process of tracking the marker on the dental instrument by using the external tracking device, the marker is highly susceptible to occlusion. Since the dental instrument is placed in the oral cavity of the patient during operation, the marker on the dental instrument that needs to be captured is highly susceptible to occlusion by a hand of the dentist or the lips of the patient, resulting in problems such as tracking loss and unstable acquisition. In addition, since a marker also needs to be provided in the oral cavity of the patient, and is usually fixed to the intraoral teeth or jawbone with adhesive, it is prone to detachment or occlusion, resulting in tracking loss, and also easily causing discomfort to the patient.

To solve the above problems, the embodiments of the present disclosure provide a method of representing the dental instrument and the reference model in the same coordinate system, which can track the dental instrument by using an intraoral scanning device instead of the external tracking device. For example, a tracking camera may be disposed on the intraoral scanning device and/or the dental instrument, and the tracking camera of the intraoral scanning device is used to capture an image of a marker on the dental instrument, and/or the tracking camera of the dental instrument is used to capture an image of a marker on the intraoral scanning device, and a real-time relative pose between the dental instrument and the intraoral scanning device is determined based on the captured images. Meanwhile, during the implant surgery, a scanning camera on the intraoral scanning device may be used to scan a target oral cavity at the same time, local data of the target oral cavity is reconstructed from the real-time image captured by the intraoral scanning device in a single scan, an alignment relationship between the local data and a pre-constructed reference model involving the target oral cavity is determined, and the reference model and the virtual dental instrument are displayed on an interactive interface based on the alignment relationship between the local data and the reference model and a relative pose relationship between the dental instrument and the intraoral scanning device, so as to indicate a pose of the dental instrument in the target oral cavity by a pose of the virtual dental instrument in the reference model.

By replacing the external tracking device with the intraoral scanning device, which is usually a portable device with a small volume, the space occupation can be greatly reduced compared with the external tracking device, thereby providing a larger working space for the medical staff. Meanwhile, the intraoral scanning device and the dental instrument both need to be placed in the target oral cavity for operation, and are positioned close to each other, thereby reducing tracking failure caused by occlusion of the markers on the intraoral scanning device or the dental instrument. In addition, with the navigation method of the present disclosure, there is no need to additionally provide markers in the target oral cavity, which can avoid tracking loss caused by detachment or occlusion of the markers, and can also reduce patient discomfort.

The navigation method provided in the embodiments of the present disclosure may be applied to a navigation system, and may be performed by various electronic devices involved in the navigation system. The electronic device may be a dental instrument, an intraoral scanning device, or another data processing apparatus communicatively connected to the dental instrument and the intraoral scanning device. The data processing apparatus may be configured to control the dental instrument to perform various operations, or the data processing apparatus may be configured to display the virtual dental instrument and the reference model to the dentist, so as to indicate the pose of the dental instrument in the target oral cavity by the pose of the virtual dental instrument in the reference model.

An execution subject of the above navigation method may be flexibly configured based on characteristics of the navigation system and specific application scenarios, and is not limited in the embodiments of the present disclosure.

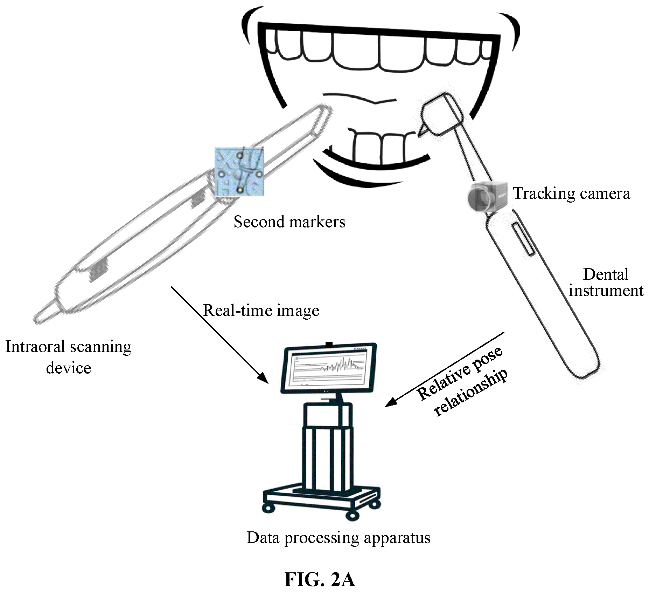

FIG. 2A is a schematic diagram of an application scenario according to an embodiment of the present disclosure, which illustrates a navigation system according to an embodiment of the present disclosure. As shown in FIG. 2A, the navigation system includes a data processing apparatus, a dental instrument, and an intraoral scanning device. The dental instrument and the intraoral scanning device may be communicatively connected to the data processing apparatus. The dental instrument is provided with a tracking camera, and a second marker is provided on a body of the intraoral scanning device, which is convenient for the dental instrument to track the intraoral scanning device. Meanwhile, the intraoral scanning device is further provided with a scanning camera for scanning and three-dimensional reconstruction of the target oral cavity.

During the implant surgery performed with the dental instrument, the tracking camera on the dental instrument may be used to track the second marker on the body of the intraoral scanning device to determine a relative pose relationship between the dental instrument and the intraoral scanning device, and send the relative pose relationship to the data processing apparatus. The intraoral scanning device may capture a real-time image of the target oral cavity, and then may send the captured real-time image to the data processing apparatus, and the data processing apparatus may reconstruct local data of the target oral cavity from the real-time image. Alternatively, the intraoral scanning device may reconstruct local data of the target oral cavity from the real-time image, and then directly send the local data of the target oral cavity to the data processing apparatus. The data processing apparatus may determine an alignment relationship between the local data of the target oral cavity and a pre-constructed reference model, and may determine respective poses of the reference model and the intraoral scanning device in a unified coordinate system based on the alignment relationship between the local data of the target oral cavity and the pre-constructed reference model. Then, the data processing apparatus may determine a pose of the dental instrument in the unified coordinate system based on the relative pose relationship between the dental instrument and the intraoral scanning device and the pose of the intraoral scanning device in the unified coordinate system. Then, the reference model and a virtual dental instrument may be displayed on an interactive interface based on the respective poses of the reference model and the dental instrument in the unified coordinate system, so as to display a real-time pose of the dental instrument in the target oral cavity by a real-time pose of the virtual dental instrument in the reference model, for the dentist to view.

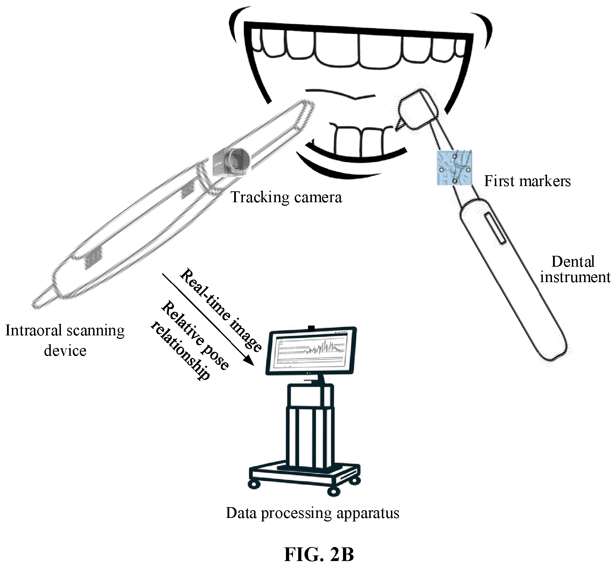

FIG. 2B is a schematic diagram of a navigation system according to another embodiment of the present disclosure. As shown in FIG. 2B, the navigation system includes a data processing apparatus, a dental instrument, and an intraoral scanning device. The intraoral scanning device is communicatively connected to the data processing apparatus. A scanning camera and a tracking camera are disposed in the intraoral scanning device, and the tracking camera may be configured to track an object. A first marker is provided on a body of the dental instrument, which is convenient for the intraoral scanning device to track the dental instrument.

During the implant surgery performed with the dental instrument, an optical tracker on the intraoral scanning device may be used to track the first marker on the body of the dental instrument to determine a relative pose relationship between the dental instrument and the intraoral scanning device, and send the relative pose relationship to the data processing apparatus. The intraoral scanning device may capture a real-time image of the target oral cavity, reconstruct local data of the target oral cavity from the real-time image, then determine an alignment relationship between the local data of the target oral cavity and a pre-constructed reference model, and based on the alignment relationship between the local data of the target oral cavity and the pre-constructed reference model, determine respective poses of the reference model and the intraoral scanning device in a unified coordinate system. Then, the data processing apparatus may determine a pose of the dental instrument in the unified coordinate system based on the relative pose relationship between the dental instrument and the intraoral scanning device and the pose of the intraoral scanning device in the unified coordinate system. Then, the reference model and a virtual dental instrument may be displayed on an interactive interface based on the respective poses of the reference model and the dental instrument in the unified coordinate system, so as to display a real-time pose of the dental instrument in the target oral cavity by a real-time pose of the virtual dental instrument in the reference model, for the dentist to view.

The reference model involving the target oral cavity in the embodiments of the present disclosure may be a CBCT (cone beam computed tomography) model obtained by CBCT, a CT model involving the target oral cavity, or a fusion model obtained by fusing the CBCT model, the CT model, and a target oral cavity model reconstructed with the intraoral scanning device or a facial scanner. The three-dimensional model may include three-dimensional information of the target oral cavity, for example, structures in the target oral cavity such as teeth, alveolar bones, blood vessels, and nerves, and may further include three-dimensional information of a face of the patient, etc.

The dental instrument in the embodiments of the present disclosure may be a tool such as a handpiece configured to drill and grind tooth structures, prepare cavities, or prepare teeth, or may be a dental instrument configured for implantation, tooth filling, restoration, veneering, and the like.

In the embodiments of the present disclosure, the intraoral scanning device may be any of various three-dimensional scanning devices configured to scan the target oral cavity, so as to reconstruct the three-dimensional model of the target oral cavity by using the scanning data captured thereby.

In the embodiments of the present disclosure, the unified coordinate system may be a coordinate system in which the reference model is located, a coordinate system of the dental instrument (that is, a coordinate system with a bur tip of the dental instrument as an origin), a coordinate system of the intraoral scanning device (a coordinate system of a camera used by the intraoral scanning device for scanning), or may be a coordinate system other than the above coordinate systems that is predefined by a user.

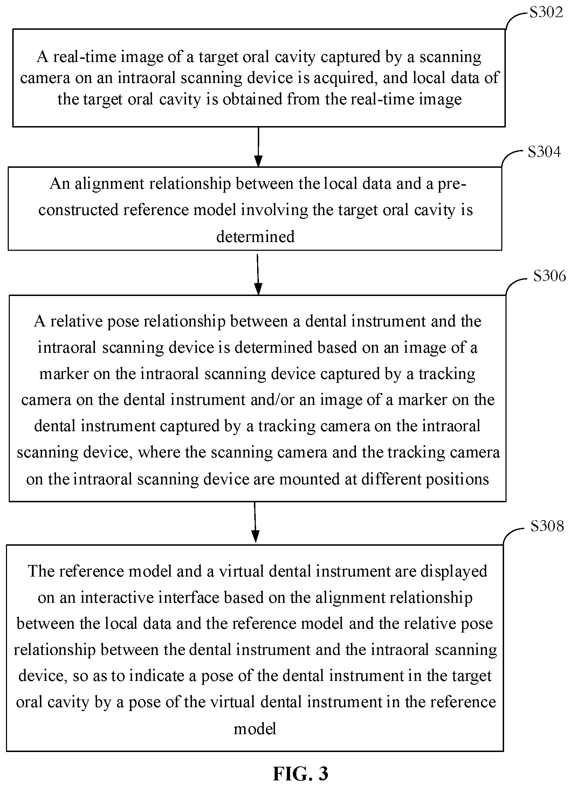

As shown in FIG. 3, the navigation method may include the following steps S302, S304, S306, and S308.

At step S302, a real-time image of a target oral cavity captured by a scanning camera on an intraoral scanning device is acquired, and local data of the target oral cavity is reconstructed from the real-time image.

In step S302, during the process of performing the implant surgery on the target oral cavity by using the dental instrument, if the dentist wants to determine the pose of the dental instrument in the target oral cavity at this time, for example, the dentist wants to determine whether the dental instrument is close to tissues such as nerves and blood vessels, the dental instrument and the pre-constructed reference model involving the target oral cavity may be registered. At this time, the dentist may place the intraoral scanning device into the target oral cavity, and scan the target oral cavity by using the intraoral scanning device. When the intraoral scanning device scans the target oral cavity, the real-time image (that is, single-frame data) currently captured by the intraoral scanning device in a single scan may be acquired, and the local data of the target oral cavity is obtained by reconstructing the target oral cavity based on the real-time image. For example, taking the intraoral scanning device including two cameras as an example, the single-frame data may include two frames of images currently captured simultaneously by the two cameras in a single image acquisition. The local data is two-dimensional data, three-dimensional data, or the like. The two-dimensional data may include a plurality of real-time images, or may be two-dimensional data obtained by processing the real-time image. Three-dimensional reconstruction is performed on the target oral cavity based on the real-time image to obtain three-dimensional data, such as a three-dimensional model, which may be a point cloud model or a mesh model.

In this way, the dentist can hold the intraoral scanning device to scan the oral cavity of the patient, so that the present disclosure can not only track the anterior teeth, but also track the posterior teeth, and there is no need to provide markers or targets in the oral cavity no matter whether the anterior teeth or the posterior teeth are tracked, which improves the comfort of the patient and reduces the steps of providing the markers or targets.

In addition, the intraoral scanning device of the present disclosure may be a monocular scanner, a binocular scanner, or a multi-ocular (with three or more cameras) scanner, which acquires real-time images by one, two, three, or more cameras, and performs reconstruction or contour tracking based on the real-time images.

At step S304, an alignment relationship between the local data and a pre-constructed reference model involving the target oral cavity is determined.

In step S304, generally, the face of the patient may be scanned by using cone beam computed tomography (CBCT) to obtain the reference model involving the target oral cavity, or the face of the patient may be scanned by using computed tomography (CT) to obtain the reference model involving the target oral cavity. The reference model may be a three-dimensional model, which includes important structures of the patient, such as teeth, gingiva, alveolar bones, nerves, and blood vessels. Since the reference model involves the entire oral cavity of the patient, there is a common area between the reference model and the local data of the target oral cavity. Therefore, the local data of the target oral cavity may be aligned with the reference model based on the information of the common area, so as to obtain the alignment relationship therebetween.

In an embodiment, the local data may be a three-dimensional model. The three-dimensional model includes geometric information of the target oral cavity, so that feature matching or stitching is performed between the three-dimensional model and the reference model based on the geometric information in the three-dimensional model, so as to determine the alignment relationship between the local data and the reference model.

In an embodiment, the local data may be a three-dimensional model, so that contour extraction is performed on the three-dimensional model of the target oral cavity to obtain contour information, and the three-dimensional model is matched with the reference model based on the contour information, so as to determine the alignment relationship between the local data and the reference model. The contour extraction may be performed by recognition using an AI model. Alternatively, a contour may be obtained by performing projection on the three-dimensional model to obtain a plurality of two-dimensional images, and then performing boundary detection on the two-dimensional images by using an algorithm such as canny. Alternatively, a contour may be obtained by performing projection on the three-dimensional model to obtain a plurality of two-dimensional images, obtaining a probability map of region detection from the plurality of two-dimensional images, and processing the probability map.

In an embodiment, the local data may be a three-dimensional model. Geometric information and contour information are obtained from the three-dimensional model, so that the three-dimensional model is matched with the reference model based on the geometric information and the contour information, thereby determining the alignment relationship between the local data and the reference model.

In some embodiments, a plurality of pieces of local data include at least one group of real-time images, and a difference in capture times among real-time images in the same group is within a preset interval. Determining the alignment relationship between the local data and the pre-constructed reference model involving the target oral cavity includes: generating a first probability map for each real-time image with a region detection algorithm, where a value of each pixel in the first probability map is configured to represent a probability that the pixel belongs to a specified tooth region; determining a viewing angle of the real-time image, and generating a second probability map for the reference model at the viewing angle based on the viewing angle of the real-time image, where a value of each pixel in the second probability map is configured to represent a probability that the pixel belongs to the specified tooth region; and determining a registration pose corresponding to a capture time period of each group of real-time images according to the first probability map and the second probability map. Based on the registration pose corresponding to the capture time period of each group of real-time images, a plurality of registration poses between the plurality of pieces of local data and the reference model may be determined, and in turn the alignment relationship between the local data and the pre-constructed reference model involving the target oral cavity may be determined.

In some embodiments, for each of the real-time images in the group (captured from different viewing angles), a region detection algorithm is used to generate an oral cavity region probability map as Probability Map Group 1 (that is, a first oral cavity probability map), where a value of each pixel represents a probability that the pixel belongs to an oral cavity region (for example, ranging from 0 to 1, where 1 indicates that the pixel definitely belongs to the oral cavity region, 0 indicates that the pixel definitely belongs to another region, and meanings of intermediate values follow the same logic). Then, the reference model is rendered onto a plurality of two-dimensional images by using a differentiable contour renderer based on a current initial pose and camera parameters, to obtain a plurality of rendered images as Probability Map Group 2 (that is, a second oral cavity probability map), where the rendered images are in a one-to-one correspondence with the viewing angles of the above real-time images, and the value of each pixel represents a probability that the pixel belongs to the oral cavity region.

The oral cavity region detected on the real-time image refers to the exposed crown region within the oral cavity, which does not include a region occluded by cheeks, gingiva, etc. Correspondingly, when the contour of the 3D oral cavity is rendered, the region occluded by the gingiva may not be rendered, which can be implemented in various ways. For example, a 3D detection mesh may be used to identify and mark the crown region and the non-crown region on the reference model, such that the non-crown region is not rendered during rendering.

In some embodiments, the initial pose for the first iteration of the first group of images may be determined by using the commonly used Perspective-n-Points (PnP) algorithm, following manual annotation of feature points on the 3D oral cavity model and the corresponding feature points in the 2D oral cavity images; and the initial pose for the subsequent iteration may be set to the result from the previous iteration. For the subsequent group of images, the pose for the first iteration may be set to the final pose tracking result from the previous group of images.

In some embodiments, the method usually regards the entire maxillary or mandibular dental arc as a single region, and constructs a regional probability map error on this basis. In practice, the method, when tracking the upper or lower jaw, can also regard each tooth (corresponding to a tooth position, where each tooth position can be automatically marked during 3D or 2D oral cavity region segmentation) as an independent region, and calculate the regional probability error separately for each tooth position, where the complete probability error is the accumulation of the probability errors of the different tooth positions. Furthermore, the cumulative probability error for individual tooth positions can even be used as an independent error term and combined with the probability error for the entire dental arc to form a stronger constraint for optimization.

In the above embodiments, the method can generate the oral cavity region probability maps for the real-time image and the global oral cavity model, respectively, and accurately determine the oral cavity registration pose corresponding to each capture time period by comparing and analyzing the two probability maps, which effectively improves the pose calculation accuracy. In turn, the alignment relationship between the local data and the pre-constructed reference model involving the target oral cavity is determined based on the registration pose.

Determining the registration pose corresponding to the capture time period of each group of real-time images according to the first probability map and the second probability map includes: accurately determining the oral registration pose corresponding to each capture time period directly based on the comparative analysis of the two probability maps, or processing the two probability maps to extract contours therefrom, and determining the oral registration pose corresponding to each capture time period based on the contour information from the two probability maps.

In some embodiments, the method further includes: processing the first probability map corresponding to each real-time image to extract a contour therefrom, and determining the plurality of registration poses between the plurality of pieces of local data and the reference model based on the contour, thereby determining the alignment relationship between the local data and the reference model.

At step S306, a relative pose relationship between the dental instrument and the intraoral scanning device is determined based on an image of a marker on the intraoral scanning device captured by a tracking camera on the dental instrument and/or an image of a marker on the dental instrument captured by a tracking camera on the intraoral scanning device, where the scanning camera and the tracking camera on the intraoral scanning device are mounted at different positions.

In step S306, in order to unify the coordinate systems of the dental instrument and the reference model, the tracking camera may be provided on the dental instrument and/or the tracking camera may be provided on the intraoral scanning device, so that the tracking camera on the dental instrument may capture the image of the marker on the intraoral scanning device and/or the intraoral scanning device may capture the image of the marker on the dental instrument, and then the relative pose relationship between the dental instrument and the intraoral scanning device may be determined based on the captured images. There may be one or more cameras provided on the dental instrument and/or the intraoral scanning device. In some embodiments, in order to obtain a more accurate three-dimensional reconstruction result, the number of the cameras may be two or more, so that depth information may be determined based on images captured by the two or more cameras from different viewing angles.

In addition, in order to perform scanning and three-dimensional reconstruction on the target oral cavity, the intraoral scanning device is further provided with the scanning camera for scanning the target oral cavity. The scanning camera and the tracking camera are located at different positions on the intraoral scanning device. For example, the scanning camera may be disposed in a housing of the intraoral scanning device and located at the front end (i.e., a scanning head) of the intraoral scanning device, and the tracking camera may be disposed at a position between a grip portion and the scanning head of the intraoral scanning device. For example, the scanning camera may be disposed in the housing of the intraoral scanning device and located at the rear end (e.g., the grip portion) of the intraoral scanning device. An acquisition window and a reflecting mirror are provided at the scanning head to collect intraoral data. The tracking camera may be disposed between the grip portion and the scanning head, and may be located on the surface of the housing of the intraoral scanning device.

The marker on the dental instrument or the intraoral scanning device may be an optical marker, a mark point carrying encoded information, a mark point without encoded information, a feature sphere, a feature pattern, or a fixing plate with a mark point or pattern, etc. The marker may be stationary. Specifically, the marker may be a fixing plate provided with a mark point or pattern, and directly fixed on the dental instrument or the intraoral scanning device, or the marker may be a mark point sticker, that is directly adhered to the dental instrument or the intraoral scanning device, or the marker may be a pattern, that is directly sprayed onto the dental instrument or the intraoral scanning device. Alternatively, the marker is movable or rotatable, so that the marker can be adjusted in time, thereby accommodating the oral cavities of different types of users (such as adults and children).

Taking the intraoral scanning device capturing the image of the dental instrument as an example, the tracking camera may be additionally provided in the intraoral scanning device, and the marker that can be observed by the tracking camera may be provided on the dental instrument. The tracking camera in the intraoral scanning device may capture images of the marker on the dental instrument from different viewing angles, and then three-dimensional reconstruction is perform on the dental instrument or the marker on the dental instrument based on the captured images, and the relative pose relationship between the intraoral scanning device and the dental instrument is determined based on the three-dimensional reconstruction result, where the marker may be a specific part or area inherent to the body of the dental instrument, or may be a target provided on the dental instrument in advance, for example, a marker provided on the dental instrument in advance.

The same applies to a scenario in which the dental instrument captures the image of the intraoral scanning device. That is, the tracking camera is additionally provided on the body of the dental instrument, the marker that can be observed by the tracking camera is provided on the intraoral scanning device, and the marker is tracked by the tracking camera, to determine a relative pose between the dental instrument and the intraoral scanning device.

At step S308, the reference model and a virtual dental instrument are displayed on an interactive interface based on the alignment relationship between the local data and the reference model and the relative pose relationship between the dental instrument and the intraoral scanning device, so as to indicate a pose of the dental instrument in the target oral cavity by a pose of the virtual dental instrument in the reference model.

In step S308, after the alignment relationship between the local data and the reference model is determined, the coordinate systems of the intraoral scanning device and the reference model may be unified based on the alignment relationship. Since the relative pose relationship between the intraoral scanning device and the dental instrument may be predetermined in various ways, the dental instrument and the reference model may be represented in the same coordinate system. Based on the poses of the dental instrument and the reference model in the same coordinate system, the reference model and the virtual dental instrument may be displayed on the interactive interface, so as to indicate the current pose of the dental instrument in the target oral cavity by the current pose of the virtual dental instrument in the reference model, which is convenient for the user to intuitively understand the condition of the dental instrument in the oral cavity.

In some embodiments, when the reference model and the virtual dental instrument are displayed on the interactive interface based on the relative pose relationship between the intraoral scanning device and the dental instrument and the alignment relationship between the local data and the reference model, so as to indicate the current pose of the dental instrument in the target oral cavity by the current pose of the virtual dental instrument in the reference model, the local data and/or the reference model may be converted to the unified coordinate system based on the alignment relationship between the local data and the reference model, so as to determine the pose of the intraoral scanning device in the unified coordinate system. For example, in some scenarios, the unified coordinate system may be a coordinate system in which the reference model is located, and the pose of the reference model in the unified coordinate system is known, so that the local data may be converted to the unified coordinate system based on the pose of the reference model in the unified coordinate system and the alignment relationship between the local data and the reference model. In some scenarios, the unified coordinate system may be a coordinate system where the intraoral scanning device is located, then the pose of the intraoral scanning device in the unified coordinate system is known, so that the reference model may be converted to the unified coordinate system based on the pose of the intraoral scanning device in the unified coordinate system and the alignment relationship between the local data and the reference model. In some scenarios, the unified coordinate system may also be a coordinate system other than the coordinate system where the reference model is located and the coordinate system where the intraoral scanning device is located, and the reference model and the intraoral scanning device need to be converted to this unified coordinate system.

After the pose of the intraoral scanning device in the unified coordinate system is determined, the pose of the dental instrument in the unified coordinate system may be determined based on the relative pose relationship between the intraoral scanning device and the dental instrument and the pose of the intraoral scanning device in the unified coordinate system. The reference model and the virtual dental instrument may be displayed on the interactive interface based on the pose of the reference model in the unified coordinate system and the pose of the dental instrument in the unified coordinate system, so as to indicate the pose of the dental instrument in the target oral cavity by the pose of the virtual dental instrument in the reference model.

In some embodiments, after the respective poses of the dental instrument and the reference model in the unified coordinate system are determined, the reference model and the virtual dental instrument may be simultaneously displayed on the interactive interface, so as to display the real-time pose of the dental instrument in the target oral cavity by the real-time pose of the virtual dental instrument in the reference model.

For example, after determining the respective poses of the dental instrument and the reference model in the unified coordinate system, the navigation system may display the reference model on the interactive interface, where the reference model involves important anatomical structures such as alveolar bones, nerves, and blood vessels. Taking the unified coordinate system being the coordinate system of the reference model as an example, the navigation system may generate a virtual dental instrument according to the pose of the dental instrument in the coordinate system of the reference model. As the dental instrument moves in the target oral cavity, the intraoral scanning device may track the dental instrument in real time and determine its real-time pose in the coordinate system of the reference model. The navigation system can reflect the pose information onto the virtual dental instrument in real time, so that the pose of the virtual dental instrument in the reference model is consistent with the pose of the dental instrument in the target oral cavity. The dentist can clearly see, from the interactive interface, the real-time position and posture of the dental instrument in the target oral cavity, and the relative relationship between the dental instrument and the surrounding anatomical structures. For example, during the actual surgical procedure, the dental instrument moves from the first tooth to the second tooth of the patient, then the virtual dental instrument on the display interface correspondingly moves from the first tooth to the second tooth in the reference model.

Generally, after obtaining the reference model, the dentist may formulate an implant surgery plan based on the positions of important structures of the patient displayed by the reference model, such as alveolar bones, nerves, and blood vessels. The movement path of the dental instrument during the implant surgery may be predetermined in the implant surgery plan, so as to avoid touching important parts such as blood vessels and nerves. Therefore, in some embodiments, after the respective poses of the dental instrument and the reference model in the unified coordinate system are determined, it may be determined whether the real-time movement path of the dental instrument in the target oral cavity deviates from the predetermined path. If the real-time movement path deviates from the predetermined path, a prompt message may be issued to prompt the dentist, thereby preventing the dental instrument from touching important parts such as blood vessels and nerves. The predetermined path is a movement path of the dental instrument in the target oral cavity as defined in the predetermined implant surgery plan.

In some embodiments, when determining the relative pose relationship between the dental instrument and the intraoral scanning device based on the image of the marker on the intraoral scanning device captured by the tracking camera on the dental instrument and/or the image of the marker on the dental instrument captured by the tracking camera on the intraoral scanning device, the following three schemes may be included.

### (1) Capturing the image of the marker on dental instrument by the tracking camera on the intraoral scanning device

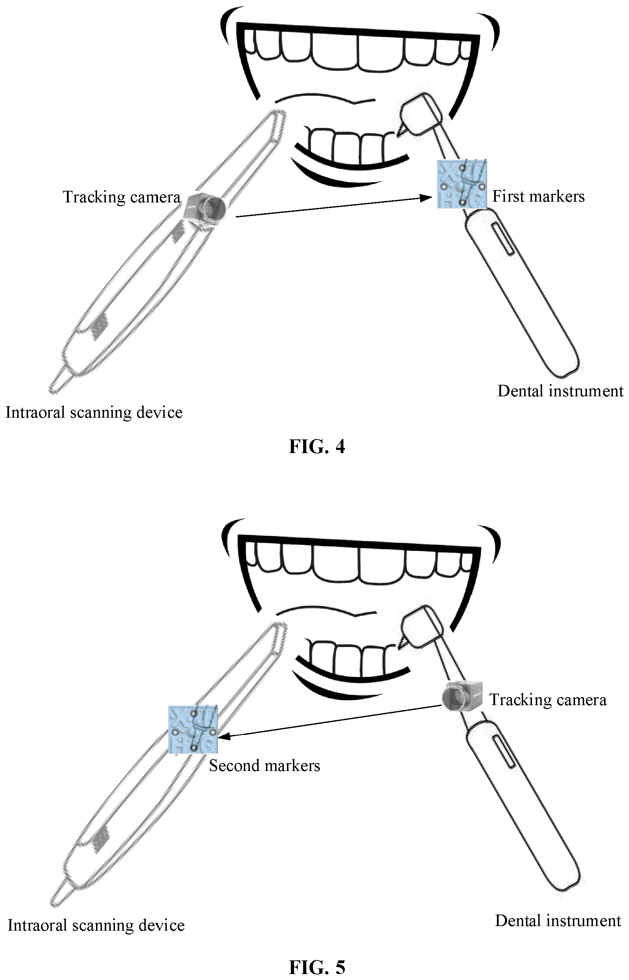

As shown in FIG. 4, the tracking camera may be additionally provided on the intraoral scanning device for tracking the dental instrument. In order to facilitate the tracking of the dental instrument, a plurality of first markers may be provided on the dental instrument, and then images of the plurality of first markers may be captured by the tracking camera on the intraoral scanning device. Three-dimensional reconstruction is performed on the plurality of first markers based on the images of the plurality of first markers to determine three-dimensional coordinates of the plurality of reconstructed first markers, and then the relative pose relationship between the intraoral scanning device and the dental instrument may be determined based on the three-dimensional coordinates of the plurality of reconstructed first markers.

### (2) Capturing the image of the marker on the intraoral scanning device by the tracking camera on the dental instrument

Similarly, as shown in FIG. 5, the tracking camera may be additionally provided on the dental instrument, while a plurality of second markers are provided on the intraoral scanning device. Images of the plurality of second markers are captured by the tracking camera on the dental instrument, then three-dimensional reconstruction may be performed on the plurality of second markers based on the images of the plurality of second markers captured by the tracking camera on the dental instrument, and the relative pose relationship between the intraoral scanning device and the dental instrument is determined based on three-dimensional coordinates of the reconstructed second markers.

### (3) The intraoral scanning device and the dental instrument capturing images of each other

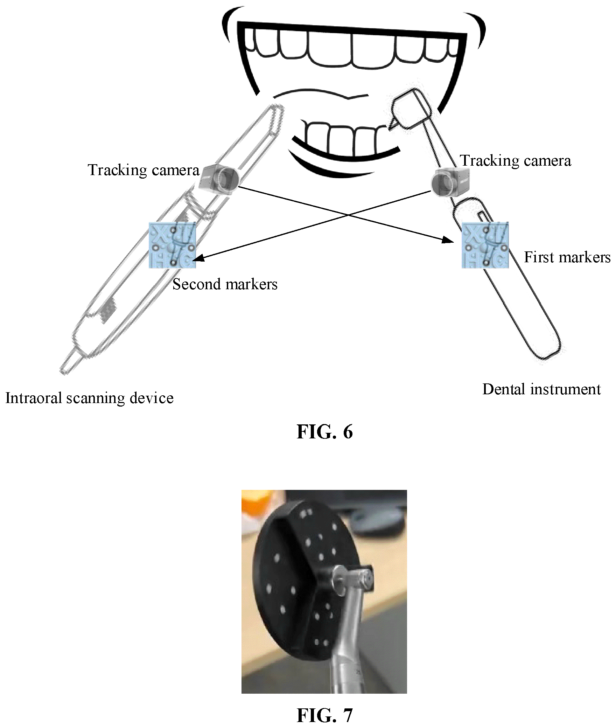

In some scenarios, as shown in FIG. 6, the tracking camera may be additionally provided on the intraoral scanning device for tracking the dental instrument, and the tracking camera may be additionally provided on the dental instrument for tracking the intraoral scanning device. At the same time, a plurality of first markers may be provided on the dental instrument, and a plurality of second markers may be provided on the intraoral scanning device, so that the intraoral scanning device and the dental instrument may capture images of each other during the implant surgery. For example, the tracking camera on the intraoral scanning device may be used to capture images of the plurality of first markers on the dental instrument, and meanwhile, the tracking camera on the dental instrument may be used to capture images of the plurality of second markers on the intraoral scanning device. Then, three-dimensional reconstruction may be performed on the plurality of first markers based on the images of the plurality of first markers captured by the intraoral scanning device, and a first pose relationship between the intraoral scanning device and the dental instrument is determined based on three-dimensional coordinates of the reconstructed first markers. Three-dimensional reconstruction may be performed on the plurality of second markers based on the images of the plurality of second markers captured by the dental instrument, and a second pose relationship between the intraoral scanning device and the dental instrument is determined based on three-dimensional coordinates of the reconstructed second markers. Then, the relative pose relationship between the intraoral scanning device and the dental instrument may be obtained based on the first pose relationship and the second pose relationship. For example, the relative pose relationship may be obtained by averaging the first pose relationship and the second pose relationship, or by weighted averaging the first pose relationship and the second pose relationship. The weight may be determined based on imaging quality of the first marker or the second marker in the images. For example, the higher the imaging quality, the greater the weight. The intraoral scanning device and the dental instrument capture images from each other, and the relative pose relationship is determined based on the images captured by the intraoral scanning device and the dental instrument, so that the tracking failure caused by occlusion of the marker can be minimized (for example, the probability that the markers on both the intraoral scanning device and the dental instrument are occluded is very low), and the accuracy of the determined relative pose relationship can be improved.

In some embodiments, an average of the first pose relationship and the second pose relationship may be determined as the relative pose relationship between the intraoral scanning device and the dental instrument.

During the surgery, dentists usually want to know a current position of an end effector (bur or needle) of the dental instrument in the target oral cavity, that is, when determining the pose of the dental instrument in the unified coordinate system, it is desired to determine the pose of the end effector of the dental instrument in the unified coordinate system. Therefore, when determining the relative pose relationship between the dental instrument and the intraoral scanning device, a relative pose relationship between the end effector of the dental instrument and the intraoral scanning device can be determined. Similarly, considering that the end effector of the dental instrument is located in the target oral cavity during the surgery, it is difficult to directly determine the relative pose relationship between the end effector of the dental instrument and the intraoral scanning device. Therefore, in some embodiments, a plurality of first markers may be provided on the body of the dental instrument. Relative pose relationships between the first markers and the end effector of the dental instrument may be pre-calibrated, for example, relative pose relationships between different types of end effectors and the first markers may be pre-calibrated and stored, and a corresponding relative pose relationship may be determined based on a type of a currently mounted end effector during use. When determining the relative pose relationship between the end effector of the dental instrument and the intraoral scanning device, the relative pose relationships between the intraoral scanning device and the first markers on the dental instrument may be determined. For example, the tracking camera on the intraoral scanning device may be used to capture images of the first markers on the dental instrument and perform three-dimensional reconstruction thereon, and the relative pose relationships between the intraoral scanning device and the first markers on the dental instrument are determined based on three-dimensional coordinates of the reconstructed first markers. Then, the relative pose relationship between the intraoral scanning device and the end effector of the dental instrument may be determined based on the pre-calibrated relative pose relationships between the first markers and the end effector of the dental instrument.

By providing the first markers on the body of the dental instrument that are readily identifiable, and pre-calibrating the relative pose relationships between the first markers and the end effector of the dental instrument, the problem that it is difficult to directly determine the relative pose between the end effector of the dental instrument and the intraoral scanning device, due to the fact that the end effector of the dental instrument is located in the oral cavity during the surgery, can be ingeniously solved.

In some embodiments, when calibrating the relative pose relationships between the first markers on the dental instrument and the end effector (e.g., a bur or a cutting tool) of the dental instrument, as shown in FIG. 7, the dental instrument without the end effector mounted may be first placed on a calibration plate, and be fixed to a central part of the calibration plate. At this time, a mounting shaft for the bur at the head of the dental instrument is arranged coaxially with an axis of the central part of the calibration plate. Then, a target scanner may be used to capture images of the dental instrument and the calibration plate and perform three-dimensional reconstruction thereon, so as to determine poses of the first markers on the dental instrument and markers on the calibration plate in the coordinate system of the target scanner. Since the relative poses between the central part of the calibration plate and the markers on the calibration plate have been determined, relative poses between the mounting shaft for the end effector (e.g., the bur or the cutting tool) at the head of the dental instrument and the first markers on the dental instrument may be determined. Next, the end effector (e.g., the bur or the cutting tool) may be mounted on the dental instrument, and the dental instrument with the end effector (e.g., the bur or the cutting tool) mounted may be placed at any position of the calibration plate (other than the center). Then, the target scanner may be used to capture images of the dental instrument and the calibration plate and perform three-dimensional reconstruction thereon, so as to determine a height (e.g., a length of the bur or the cutting tool) of the end effector of the dental instrument, so that relative poses between the end effector of the dental instrument (e.g., the axis and length of the bur or the cutting tool) and the first markers may be obtained. The target scanner may be a scanner with a relatively large imaging field of view, for example, a facial scanner. Alternatively, the target scanner may be a high-precision optical tracker, which is capable of capturing and tracking position and posture information of a plurality of target objects in real time. The optical tracker determines three-dimensional coordinates of the target objects by emitting and receiving optical signals (e.g., infrared light).

Since the local data of the target oral cavity is a three-dimensional model in the coordinate system of the scanning camera of the intraoral scanning device, that is, the determined pose of the intraoral scanning device in the unified coordinate system is substantially the pose of the scanning camera of the intraoral scanning device in the unified coordinate system, determining the relative pose relationship between the dental instrument and the intraoral scanning device may be equivalent to determining the relative pose relationship between the dental instrument and the scanning camera on the intraoral scanning device, so that the pose of the dental instrument in the unified coordinate system can be accurately determined. Considering that the scanning camera of the intraoral scanning device is often located in the oral cavity during the surgery, it is difficult to track the scanning camera to directly determine the relative pose relationship between the dental instrument and the scanning camera of the intraoral scanning device. Therefore, in some embodiments, a plurality of second markers may be provided on the body of the intraoral scanning device. The relative poses between the second markers and the scanning camera of the intraoral scanning device may be pre-calibrated. When the relative pose relationship between the dental instrument and the scanning camera of the intraoral scanning device is determined, the tracking camera on the dental instrument may be used to capture images of the second markers and perform three-dimensional reconstruction thereon, and relative pose relationships between the dental instrument and the second markers on the intraoral scanning device are determined based on three-dimensional coordinates of the reconstructed second markers. Then, the relative pose relationship between the scanning camera on the intraoral scanning device and the dental instrument is determined based on the pre-calibrated relative pose relationships between the second markers and the scanning camera on the intraoral scanning device.

By providing the second markers on the body of the intraoral scanning device that are readily identifiable and pre-calibrating the relative pose relationships between the second markers and the scanning camera on the intraoral scanning device, the problem that it is difficult to directly determine the relative pose between the dental instrument and the scanning camera on the intraoral scanning device during the surgery is ingeniously solved. By determining the relative pose relationships between the dental instrument and the second markers, and then indirectly determining the relative pose relationship between the dental instrument and the scanning camera of the intraoral scanning device by using the pre-calibrated relative pose relationships between the second markers and the scanning camera, accurate registration between the reference model and the dental instrument can be achieved. This method not only effectively overcomes the problem that the scanning camera or the acquisition window of the intraoral scanning device is located in the oral cavity and is difficult to be directly tracked during the scanning, but also improves the accuracy and reliability of registration, thereby ensuring the accuracy of the implant surgery navigation.

In some embodiments, in order to accurately calibrate the relative pose relationships between the second markers on the body of the intraoral scanning device and the scanning camera on the intraoral scanning device, a calibration member may be used to calibrate the two. The calibration member may include a plurality of markers with known relative pose relationships. During the calibration process, the scanning camera of the intraoral scanning device may be used to capture an image of the calibration member and perform three-dimensional reconstruction thereon to obtain a first calibration model, where the first calibration model includes at least part of markers on the calibration member. Meanwhile, the target scanner may be used to capture images of the intraoral scanning device and the calibration member and perform three-dimensional reconstruction thereon to obtain a second calibration model, where the second calibration model includes at least part of the second markers and at least part of the markers on the calibration member. Since the first calibration model is a model in the coordinate system of the scanning camera of the intraoral scanning device, and the second calibration model is a model in the coordinate system of the target scanner, the coordinate systems of the first calibration model and the second calibration model may be unified based on at least part of the markers on the calibration member, so as to determine the relative pose relationships between the second markers and the scanning camera of the intraoral scanning device. The target scanner may be a scanner with a relatively large imaging field of view, for example, a facial scanner.

Sizes, shapes, and quantities of the first marker and the second marker may be set based on actual requirements, and are not limited in the embodiments of the present disclosure. In some scenarios, the first marker and the second marker may be encoded points carrying encoded information, to facilitate distinguishing the plurality of first markers based on the encoded information, or distinguishing the plurality of second markers based on the encoded information. In some embodiments, relative positional relationships between the plurality of first markers on the dental instrument are known, and relative positional relationships between the plurality of second markers on the intraoral scanning device are known.

In some embodiments, in order to facilitate the tracking camera of the intraoral scanning device to capture the image of the first marker on the dental instrument and reduce the probability that the first marker is occluded, the position of the first marker may be set based on the position of the tracking camera on the intraoral scanning device, that is, the first marker may be provided at a position convenient for the tracking camera on the intraoral scanning device to observe.

Similarly, in order to facilitate the tracking camera on the dental instrument to capture the image of the second marker on the intraoral scanning device and reduce the probability that the second marker is occluded, the position of the second marker may be set based on the position of the tracking camera on the dental instrument, that is, the second marker may be provided at a position convenient for the tracking camera on the dental instrument to observe.

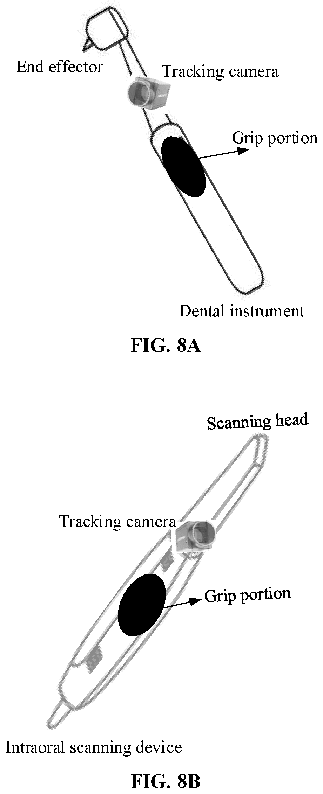

In some embodiments, as shown in FIG. 8A, the dental instrument is provided with a grip portion, the tracking camera on the dental instrument is disposed at a position between the grip portion of the dental instrument and the end effector of the dental instrument, and the marker on the intraoral scanning device is disposed at a position observable by the tracking camera on the dental instrument.

In some embodiments, as shown in FIG. 8B, the intraoral scanning device is provided with a grip portion, the tracking camera on the intraoral scanning device is disposed at a position between the grip portion of the intraoral scanning device and a scanning head of the intraoral scanning device, and the marker on the dental instrument is disposed at a position observable by the tracking camera on the intraoral scanning device.

By arranging the tracking camera at a position between the grip portion and the end effector of the dental instrument, or at a position between the grip portion of the intraoral scanning device and the scanning head of the intraoral scanning device, the camera or the marker can be prevented from being occluded during the surgery, so that the tracking camera can accurately track the marker.

In some embodiments, when the local data of the target oral cavity is aligned with the pre-constructed reference model involving the target oral cavity to determine the alignment relationship therebetween, the alignment relationship may be determined directly based on a common feature between the local data of the target oral cavity and the reference model. For example, registration may be performed on a common area between the local data of the target oral cavity and the reference model to determine the alignment relationship therebetween.

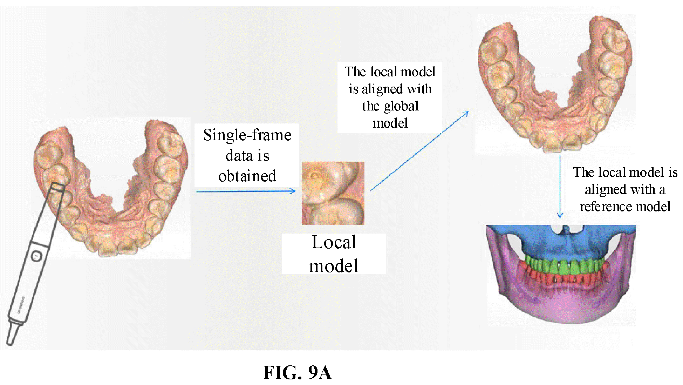

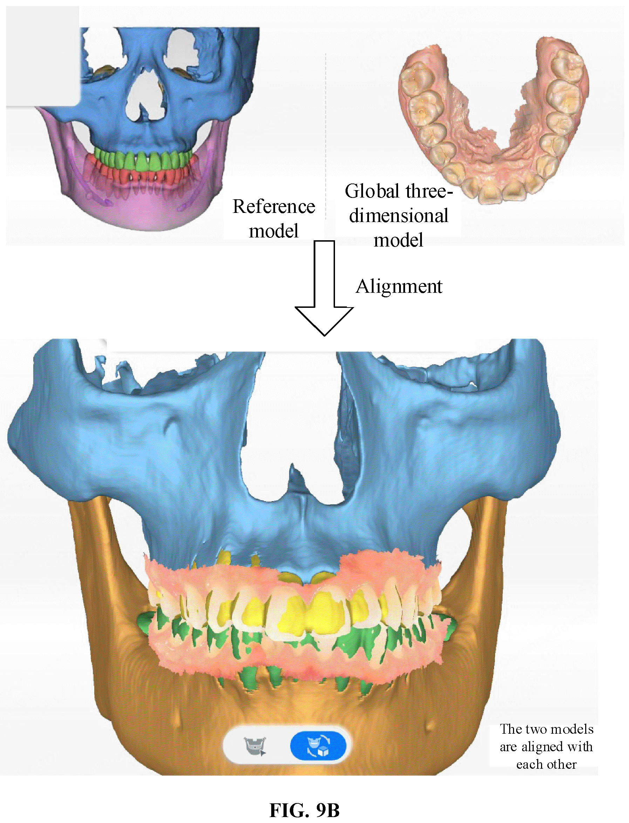

In some embodiments, considering that the local data of the target oral cavity covers only a small area of the target oral cavity, the common area between the local data and the reference model is limited, and directly aligning the local data and the reference model would result in relatively low accuracy. In order to improve the accuracy of the alignment relationship between the local data and the reference model, as shown in FIG. 9A, the intraoral scanning device may be first used to scan the target oral cavity, to construct a global three-dimensional model of the target oral cavity. Then, as shown in FIG. 9B, the relationship between the reference model and the global three-dimensional model of the target oral cavity may be determined based on common features between the reference model and the pre-constructed global three-dimensional model of the target oral cavity, and the alignment relationship between the local data of the target oral cavity and the global three-dimensional model of the target oral cavity may be determined based on common features between the local data of the target oral cavity (for example, the local three-dimensional model of the target oral cavity in the drawings) and the global three-dimensional model of the target oral cavity. Then, the alignment relationship between the reference model and the local data may be determined based on the alignment relationship between the reference model and the global three-dimensional model of the target oral cavity and the alignment relationship between the local data of the target oral cavity and the global three-dimensional model of the target oral cavity.

The global three-dimensional model constructed by the intraoral scanning device covers a wider oral cavity area, and provides richer common features for alignment with the reference model, thereby enhancing the accuracy and reliability of the alignment. In addition, since the local data of the target oral cavity and the global three-dimensional model of the target oral cavity are captured by the same intraoral scanning device, the alignment between the two can be achieved with higher accuracy. This stepwise alignment method avoids the problem of insufficient accuracy that would otherwise arise from directly aligning the local data of the target oral cavity with the reference model due to the limited common areas therebetween.

In some embodiments, the common feature includes an inherent feature in the target oral cavity, where the inherent feature includes one or more of teeth, gingiva, and palate of the patient. Since the teeth, gingiva, palate, and the like in the target oral cavity can be used as common features for aligning the local data of the target oral cavity with the reference model, or as common features for aligning the local data of the target oral cavity with the global three-dimensional model of the target oral cavity, the local data of the target oral cavity can be aligned with the reference model or the local data of the target oral cavity can be aligned with the global three-dimensional model of the target oral cavity directly based on the inherent features in the target oral cavity, so that there is no need to additionally affix reference marks in the target oral cavity, which can simplify the operation procedure in the implant navigation surgery and reduce the discomfort of the patient.

In some embodiments, the common feature includes a reference mark additionally provided in the target oral cavity. Considering that some patients may have lost most of their teeth, that is, there may be relatively few inherent features in the target oral cavity, or these inherent features may not be readily identified and distinguished, the alignment accuracy may be reduced. In this scenario, in order to improve the accuracy of aligning the local data of the target oral cavity with the reference model or the accuracy of aligning the local data of the target oral cavity with the global three-dimensional model of the target oral cavity, the reference mark may be additionally provided in the oral cavity of the patient. The reference mark may include readily identifiable markers such as feature points, so that the alignment of the local data with the reference model or the alignment of the local data of the target oral cavity with the global three-dimensional model of the target oral cavity may be realized based on these markers.

In some embodiments, in order to provide richer common features, the common features may include both the inherent features in the target oral cavity and the above reference marks, so as to improve the alignment accuracy.

In some embodiments, there are a plurality of types of common features. For example, the common features may include reference marks provided in the oral cavity of the patient, teeth, gingiva, palate, and the like. In order to improve the alignment accuracy between the reference model and the local data of the target oral cavity, the plurality of types of common features may be used for registration. For example, when the alignment relationship between the reference model and the local data of the target oral cavity is determined based on the common feature information in the local data and the reference model, a first alignment relationship between the reference model and the local data of the target oral cavity may be determined for each type of common feature, based on the registration relationship between that type of common feature in the local data and the corresponding type of common feature in the reference model. Then, weighted averaging is performed on first alignment relationships respectively corresponding to the plurality of types of common features based on weights respectively corresponding to the plurality of types of common features, so as to obtain the alignment relationship between the reference model and the local data. Considering that different types of common features differ in reliability and accuracy, different weights are assigned to the common features according to their importance when calculating the final relative pose relationship. A magnitude relationship between the weights respectively corresponding to the plurality of types of common features is as follows: a weight of the reference mark is greater than a weight of the tooth of the patient, the weight of the tooth of the patient is greater than a weight of the gingiva of the patient, and the weight of the gingiva of the patient is greater than a weight of the palate of the patient.

Considering that the reference marks provided in the oral cavity of the patient are high-precision feature points, they are commonly used for precise alignment. By using the reference marks for registration, a high-precision relative pose relationship can be obtained, so the reference marks are assigned the highest weight. The morphology and position of the teeth exhibit good consistency between the reference model and the three-dimensional model of the target oral cavity, and thus the teeth can be used as reliable registration features, so the teeth are assigned a weight slightly lower than that of the reference marks. The morphology of the gingiva can also be well matched between the reference model and the scan model of the target oral cavity, but its accuracy is slightly lower than those of the teeth and the reference marks. The morphology of the palate can also be used for registration, but its accuracy is relatively low, so it is assigned the smallest weight.

For example, the registration of the local data of the target oral cavity with the reference model is taken as an example. Based on the registration relationship of the reference mark provided in the oral cavity, it can be determined that the relative pose relationship between the local data of the target oral cavity and the reference model is R1. Based on the teeth in the target oral cavity, it can be determined that the relative pose relationship between the local data of the target oral cavity and the reference model is R2. Based on the gingiva in the target oral cavity, it can be determined that the relative pose relationship between the local data of the target oral cavity and the reference model is R3. The weights of the reference mark, the teeth, and the gingiva are w1, w2, and w3, respectively, where w1> w2> w3. Finally, it can be determined that the relative pose relationship between the local data of the target oral cavity and the reference model is: R = w1R1+ w2R2+ w3R3.

By combining a plurality of types of common features for registration and assigning different weights according to the reliability of each type of common feature, the accuracy and reliability of different features can be comprehensively considered, thereby improving the accuracy of the determined relative pose relationship between the reference model and the intraoral scanning device. This method not only makes full use of the advantages of various types of features, but also reduces potential errors that may be introduced by any single feature through weighted averaging, thereby improving accuracy and reliability of surgical navigation.

In some embodiments, the alignment relationship between the global three-dimensional model (or local data) of the target oral cavity and the reference model may be determined in the following manner. Semantic segmentation may be performed on teeth in the global three-dimensional model (or local data) to obtain individual teeth in the global three-dimensional model (or local data). Similarly, semantic segmentation may be performed on teeth in the reference model to obtain individual teeth in the reference model. Then, the teeth in the global three-dimensional model (or local data) may be aligned with the teeth in the reference model based on the tooth position numbers of the individual teeth. For example, the teeth with the same tooth position number are aligned, and then the alignment relationship between the global three-dimensional model (or local data) and the reference model may be determined.

In addition, the embodiments of the present disclosure further provide a navigation system, including a dental instrument, an intraoral scanning device, and a data processing apparatus, where the data processing apparatus is communicatively connected to the intraoral scanning device, and the intraoral scanning device is configured to capture a real-time image of a target oral cavity by using a scanning camera, and send the real-time image to the data processing apparatus.

The data processing apparatus is configured to: acquire the real-time image, and reconstruct local data of the target oral cavity from the real-time image; determine an alignment relationship between the local data and a pre-constructed reference model involving the target oral cavity; determine a relative pose relationship between the dental instrument and the intraoral scanning device based on an image of a marker on the intraoral scanning device captured by a tracking camera on the dental instrument and/or an image of a marker on the dental instrument captured by a tracking camera on the intraoral scanning device, where the scanning camera and the tracking camera on the intraoral scanning device are mounted at different positions; and display the reference model and a virtual dental instrument on an interactive interface based on the alignment relationship between the local data and the reference model and the relative pose relationship between the dental instrument and the intraoral scanning device, so as to indicate a pose of the dental instrument in the target oral cavity by a pose of the virtual dental instrument in the reference model.

For specific functions of the navigation system, reference may be made to the descriptions in the above embodiments, which will not be repeated herein.

It should be readily understood that the solutions described in the above embodiments can be freely combined without conflict to obtain new solutions, which are not enumerated in the embodiments of the present disclosure for the sake of brevity.

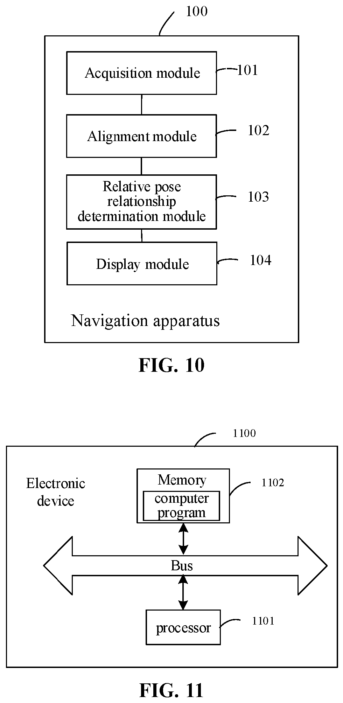

Correspondingly, the embodiments of the present disclosure further provide a navigation apparatus. As shown in FIG. 10, the apparatus 100 includes an acquisition module 101, an alignment module 102, a relative pose relationship determination module 103, and a display module 104.

The acquisition module 101 is configured to acquire a real-time image of a target oral cavity captured by a scanning camera on an intraoral scanning device, and reconstruct local data of the target oral cavity from the real-time image.

The alignment module 102 is configured to determine an alignment relationship between the local data and a pre-constructed reference model involving the target oral cavity.

The relative pose relationship determination module 103 is configured to determine a relative pose relationship between a dental instrument and the intraoral scanning device based on an image of a marker on the intraoral scanning device captured by a tracking camera on the dental instrument and/or an image of a marker on the dental instrument captured by a tracking camera on the intraoral scanning device, where the scanning camera and the tracking camera on the intraoral scanning device are mounted at different positions.

The display module 104 is configured to display the reference model and a virtual dental instrument on an interactive interface based on the alignment relationship between the local data and the reference model and the relative pose relationship between the dental instrument and the intraoral scanning device, so as to indicate a pose of the dental instrument in the target oral cavity by a pose of the virtual dental instrument in the reference model.

For the specific steps of performing the navigation method by the above apparatus, reference may be made to the descriptions in the above method embodiments, which will not be repeated herein.

Further, the embodiments of the present disclosure further provide an electronic device. As shown in FIG. 11, the electronic device 1100 includes at least one processor 1101, at least one memory 1102, and computer instructions stored in the memory 1102 and executable by the processor 1101, where the processor 1101, when executing the computer instructions, implements the method according to any one of the above embodiments.

The embodiments of the present disclosure further provide a computer-readable storage medium, having a computer program stored therein, where the computer program, when executed by at least one processor, causes the method according to any one of the foregoing embodiments to be implemented.

The computer-readable medium includes permanent and non-permanent, and removable and non-removable media that can implement information storage by any method or technology. The information may be a computer-readable instruction, a data structure, a program module, or other data. Examples of a storage medium of a computer include, but are not limited to, a phase change random access memory (PRAM), a static random access memory (SRAM), a dynamic random access memory (DRAM), another type of random access memory (RAM), a read-only memory (ROM), an electrically erasable programmable read-only memory (EEPROM), a flash memory, or another memory technology, a compact disc read-only memory (CD-ROM), a digital versatile disc (DVD) or another optical storage, a magnetic cassette tape, a magnetic disk storage, or another magnetic storage device, or any other non-transmission medium, which can be used to store information that can be accessed by a computing device. As defined herein, the computer-readable medium does not include transitory computer-readable media, such as modulated data signals and carriers.

It can be learned from the foregoing descriptions of the implementations that those skilled in the art may clearly understand that the embodiments of the present disclosure may be implemented by means of software in addition to a necessary universal hardware platform. Based on such understanding, the technical solutions of the embodiments of the present disclosure essentially or the part contributing to the prior art may be embodied in the form of a software product, and the computer software product may be stored in a storage medium, such as an ROM/RAM, a magnetic disk, an optical disk, etc., and includes several instructions to enable a computer device (which may be a personal computer, a server, or a network device, etc.) to perform the methods according to various embodiments or some parts of the embodiments of the present disclosure.

The system, apparatus, module, or unit described in the foregoing embodiments may be implemented by a computer chip or an entity, or may be implemented by a product having a certain function. A typical implementation device is a computer, and a specific form of the computer may be a personal computer, a laptop computer, a cellular phone, a camera phone, a smartphone, a personal digital assistant, a media player, a navigation apparatus, an email transceiver device, a game console, a tablet computer, a wearable device, or a combination of any two or more of these devices.

The various embodiments in this specification are described in a progressive manner, the same or similar parts between the various embodiments can be referred to each other, and each embodiment focuses on the differences from other embodiments. In particular, for the apparatus embodiment, since it is basically similar to the method embodiment, the description thereof is relatively simple, and the relevant parts can be referred to the description of the method embodiment. The apparatus embodiments described above are merely illustrative, where modules described as separate components may or may not be physically separated, and functions of the modules may be implemented in one or more of software and/or hardware when the solutions of the embodiments of the present disclosure are implemented. Some or all of the modules may also be selected according to actual needs to achieve the objectives of the solutions of the embodiments. Those skilled in the art can understand and implement without creative effort.

The above are only specific implementations of the embodiments of the present disclosure, and it should be noted that for those of ordinary skill in the art, several improvements and modifications can be made without departing from the principles of the embodiments of the present disclosure, and these improvements and modifications should also be considered to be within the scope of protection of the embodiments of the present disclosure.

## Claims

1. A navigation method, comprising:
acquiring a real-time image of a target oral cavity captured by a scanning camera on an intraoral scanning device, and reconstructing local data of the target oral cavity from the real-time image;
determining an alignment relationship between the local data and a pre-constructed reference model involving the target oral cavity;
determining a relative pose relationship between a dental instrument and the intraoral scanning device based on an image of a marker on the intraoral scanning device captured by a tracking camera on the dental instrument and/or an image of a marker on the dental instrument captured by a tracking camera on the intraoral scanning device, wherein the scanning camera and the tracking camera on the intraoral scanning device are mounted at different positions; and
displaying the reference model and a virtual dental instrument on an interactive interface based on the alignment relationship between the local data and the reference model and the relative pose relationship between the dental instrument and the intraoral scanning device, so as to indicate a pose of the dental instrument in the target oral cavity by a pose of the virtual dental instrument in the reference model.

2. The method according to claim 1, wherein determining the alignment relationship between the local data and the pre-constructed reference model involving the target oral cavity comprises:
determining geometric information and/or contour information of the target oral cavity according to the local data; and
determining the alignment relationship between the local data of the target oral cavity and the reference model involving the target oral cavity based on the geometric information and/or the contour information of the target oral cavity.

3. The method according to claim 1 or 2, further comprising:
in response to determining, based on the alignment relationship between the local data and the reference model and the relative pose relationship between the dental instrument and the intraoral scanning device, that a real-time movement path of the dental instrument in the target oral cavity deviates from a predetermined path, issuing a prompt message, wherein the predetermined path is a movement path of the dental instrument in the target oral cavity as defined in a predetermined implant plan.

4. The method according to any one of claims 1 to 3, wherein determining the relative pose relationship between the dental instrument and the intraoral scanning device based on the image of the marker on the intraoral scanning device captured by the tracking camera on the dental instrument and/or the image of the marker on the dental instrument captured by the tracking camera on the intraoral scanning device comprises:
in the case where the marker on the dental instrument comprises a plurality of first markers provided on a body of the dental instrument, performing three-dimensional reconstruction on the plurality of first markers based on images of the plurality of first markers captured by the tracking camera on the intraoral scanning device, and determining the relative pose relationship between the dental instrument and the intraoral scanning device based on three-dimensional coordinates of reconstructed first markers; or
in the case where the marker on the intraoral scanning device comprises a plurality of second markers provided on a body of the intraoral scanning device, performing three-dimensional reconstruction on the plurality of second markers based on images of the plurality of second markers captured by the tracking camera on the dental instrument, and determining the relative pose relationship between the dental instrument and the intraoral scanning device based on three-dimensional coordinates of reconstructed second markers; or
in the case where the marker on the dental instrument comprises a plurality of first markers provided on a body of the dental instrument, and the marker on the intraoral scanning device comprises a plurality of second markers provided on a body of the intraoral scanning device, performing three-dimensional reconstruction on the plurality of first markers based on images of the plurality of first markers captured by the tracking camera on the intraoral scanning device, and determining a first pose relationship between the dental instrument and the intraoral scanning device based on three-dimensional coordinates of reconstructed first markers; performing three-dimensional reconstruction on the plurality of second markers based on images of the plurality of second markers captured by the tracking camera on the dental instrument, and determining a second pose relationship between the dental instrument and the intraoral scanning device based on three-dimensional coordinates of reconstructed second markers; and obtaining the relative pose relationship between the dental instrument and the intraoral scanning device based on the first pose relationship and the second pose relationship.

5. The method according to claim 4, wherein obtaining the relative pose relationship between the dental instrument and the intraoral scanning device based on the first pose relationship and the second pose relationship comprises:
determining an average of the first pose relationship and the second pose relationship as the relative pose relationship between the dental instrument and the intraoral scanning device.

6. The method according to claim 4, wherein the relative pose relationship between the dental instrument and the intraoral scanning device is a relative pose relationship between an end effector on the dental instrument and the intraoral scanning device; and determining the relative pose relationship between the dental instrument and the intraoral scanning device based on the three-dimensional coordinates of the reconstructed first markers comprises:
determining a relative pose relationship between the intraoral scanning device and the first markers based on the three-dimensional coordinates of the reconstructed first markers; and
determining the relative pose relationship between the end effector and the intraoral scanning device based on a pre-calibrated relative pose relationship between the first markers and the end effector and the relative pose relationship between the intraoral scanning device and the first markers.

7. The method according to claim 4, wherein the relative pose relationship between the dental instrument and the intraoral scanning device is a relative pose relationship between the dental instrument and the scanning camera on the intraoral scanning device; and determining the relative pose relationship between the dental instrument and the intraoral scanning device based on the three-dimensional coordinates of the reconstructed second markers comprises:
determining a relative pose relationship between the dental instrument and the second markers based on the three-dimensional coordinates of the reconstructed second markers; and
determining the relative pose relationship between the dental instrument and the scanning camera on the intraoral scanning device based on a pre-calibrated relative pose relationship between the second markers and the scanning camera on the intraoral scanning device and the relative pose relationship between the dental instrument and the second markers.

8. The method according to claim 7, wherein the relative pose relationship between the second markers and the scanning camera on the intraoral scanning device is determined by:
capturing an image of a calibration member by the intraoral scanning device and performing three-dimensional reconstruction to obtain a first calibration model, wherein the first calibration model comprises at least part of markers on the calibration member;
capturing images of the intraoral scanning device and the calibration member by a target scanner and performing three-dimensional reconstruction to obtain a second calibration model, wherein the second calibration model comprises at least part of the second markers and at least part of the markers on the calibration member;
unifying a coordinate system of the first calibration model and a coordinate system of the second calibration model based on at least part of the markers on the calibration member to determine the relative pose relationship between the second markers and the scanning camera on the intraoral scanning device.

9. The method according to any one of claims 1 to 8, wherein
the dental instrument is provided with a grip portion, the tracking camera on the dental instrument is disposed at a position between the grip portion of the dental instrument and an end effector of the dental instrument, and the marker on the intraoral scanning device is disposed at a position observable by the tracking camera on the dental instrument; and/or
the intraoral scanning device is provided with a grip portion, the tracking camera on the intraoral scanning device is disposed at a position between the grip portion of the intraoral scanning device and a scanning head of the intraoral scanning device, and the marker on the dental instrument is disposed at a position observable by the tracking camera on the intraoral scanning device.

10. The method according to any one of claims 1 to 9, wherein determining the alignment relationship between the local data and the reference model involving the target oral cavity comprises:
determining the alignment relationship between the local data and the reference model based on a common feature between the local data and the reference model; or
determining an alignment relationship between the reference model and a pre-constructed global three-dimensional model of the target oral cavity based on a common feature between the reference model and the global three-dimensional model; determining an alignment relationship between the local data and the global three-dimensional model based on a common feature between the local data and the global three-dimensional model; and determining the alignment relationship between the local data and the reference model based on the alignment relationship between the reference model and the global three-dimensional model and the alignment relationship between the local data and the global three-dimensional model, wherein the global three-dimensional model is reconstructed based on images of the target oral cavity captured by the intraoral scanning device.

11. The method according to any one of claims 1 to 10, wherein displaying the reference model and the virtual dental instrument on the interactive interface based on the alignment relationship between the local data and the reference model and the relative pose relationship between the dental instrument and the intraoral scanning device comprises:
converting the local data and/or the reference model to a unified coordinate system based on the alignment relationship between the local data and the reference model, to determine a pose of the intraoral scanning device in the unified coordinate system;
determining a pose of the dental instrument in the unified coordinate system based on the pose of the intraoral scanning device in the unified coordinate system and the relative pose relationship between the dental instrument and the intraoral scanning device; and
displaying the reference model and the virtual dental instrument on the interactive interface based on the pose of the dental instrument in the unified coordinate system and a pose of the reference model in the unified coordinate system, so as to indicate the pose of the dental instrument in the target oral cavity by the pose of the virtual dental instrument in the reference model.

12. The method according to any one of claims 1 to 11, wherein the local data comprises at least one group of real-time images, and a difference in capture times among real-time images in a same group is within a preset interval;
wherein determining the alignment relationship between the local data and the pre-constructed reference model involving the target oral cavity comprises:
generating a first probability map for each of the real-time images with a region detection algorithm, wherein a value of each pixel in the first probability map is configured to represent a probability that the pixel belongs to a specified tooth region;
determining a viewing angle of the real-time image, and generating a second probability map for the reference model at the viewing angle based on the viewing angle of the real-time image, wherein a value of each pixel in the second probability map is configured to represent a probability that the pixel belongs to the specified tooth region;
determining, according to the first probability map and the second probability map, a registration pose corresponding to a capture time period of each group of real-time images; and
determining the alignment relationship between the local data and the pre-constructed reference model involving the target oral cavity based on the registration pose.

13. A navigation system, comprising a dental instrument, an intraoral scanning device, and a data processing apparatus communicatively connected to the intraoral scanning device; wherein
the intraoral scanning device is configured to capture a real-time image of a target oral cavity by a scanning camera, and send the real-time image to the data processing apparatus; and
the data processing apparatus is configured to: acquire the real-time image, and reconstruct local data of the target oral cavity from the real-time image; determine an alignment relationship between the local data and a pre-constructed reference model involving the target oral cavity; determine a relative pose relationship between the dental instrument and the intraoral scanning device based on an image of a marker on the intraoral scanning device captured by a tracking camera on the dental instrument and/or an image of a marker on the dental instrument captured by a tracking camera on the intraoral scanning device, wherein the scanning camera and the tracking camera on the intraoral scanning device are mounted at different positions; and display the reference model and a virtual dental instrument on an interactive interface based on the alignment relationship between the local data and the reference model and the relative pose relationship between the dental instrument and the intraoral scanning device, so as to indicate a pose of the dental instrument in the target oral cavity by a pose of the virtual dental instrument in the reference model.

14. A navigation apparatus, comprising:
an acquisition module, configured to acquire a real-time image of a target oral cavity captured by a scanning camera on an intraoral scanning device, and reconstruct local data of the target oral cavity from the real-time image;
an alignment module, configured to determine an alignment relationship between the local data and a pre-constructed reference model involving the target oral cavity;
a relative pose relationship determination module, configured to determine a relative pose relationship between a dental instrument and the intraoral scanning device based on an image of a marker on the intraoral scanning device captured by a tracking camera on the dental instrument and/or an image of a marker on the dental instrument captured by a tracking camera on the intraoral scanning device, wherein the scanning camera and the tracking camera on the intraoral scanning device are mounted at different positions; and
a display module, configured to display the reference model and the virtual dental instrument on an interactive interface based on the alignment relationship between the local data and the reference model and the relative pose relationship between the dental instrument and the intraoral scanning device, so as to indicate a pose of the dental instrument in the target oral cavity by a pose of the virtual dental instrument in the reference model.

15. An electronic device, comprising at least one processor, at least one memory, and computer instructions stored in the memory and executable by the processor, wherein the processor, when executing the computer instructions, implements the method according to any one of claims 1 to 12.
